# EUROPEAN PATENT APPLICATION

(11) **EP 3 851 843 A1**
(43) Date of publication of application: **21.07.2021**
(21) Application number: 18933008.7
(22) Date of filing: 13.09.2018
(51) Int. Cl.: G01N 27/28

(54) **NUCLEOTIDE SEQUENCING ELEMENT AND CHIP, AND SEQUENCING ANALYSIS METHOD**

(71) Applicant: Lien, Chun-Lung, Hsinchu County 308, Taiwan (TW); Shiau, Jeng-Huei, Zhubei City, Hsinchu County 302, Taiwan (TW)
(72) Inventor: Lien, Chun-Lung, Hsinchu County 308, Taiwan (TW); Shiau, Jeng-Huei, Zhubei City, Hsinchu County 302, Taiwan (TW)
(74) Representative: Cabinet Beaumont
(86) International application number: PCT/CN2018/105428
(87) International publication number: WO 2020/051824

(57) **Abstract**

Provided is a nucleotide sequencing element, comprising a semiconductor substrate, a transistor, a dielectric layer, an annular electrode group, and a conductor. Also provided is a nucleotide sequencing chip, comprising the nucleotide sequencing element and a sense amplifier. Also provided is a sequencing analysis method, comprising: providing the nucleotide sequencing chip as stated above; placing a nucleotide sequence fragment to be tested and a known nucleotide sequence fragment in a slot separately for a polymerization reaction and an electrochemical reaction; and analyzing the electrical signals generated by the reactions to obtain a sequencing result.

## Description

### BACKGROUND

### Field of Invention

The present disclosure relates to a sequencing element, a chip, and a method for sequencing analysis. More particularly, the present disclosure relates to an element and a chip for nucleotide sequencing, and a method for nucleotide sequencing analysis.

### Description of Related Art

Deoxyribonucleic acid (DNA) sequencing refers to the analysis of the base sequence of specific DNA fragments, that is, the analysis of adenine (A), thymine (T), cytosine (C) and guanine (G) arrangement. DNA sequencing has been widely used, and the scope is roughly divided into six major projects: basic research (gene structure, gene function), genome research, origin of life, race or species differences, specific gene variations and diseases, and test reagents with new drug development.

Recently, DNA sequencing has been improved to next-generation sequencing, one of which is a sequencing method based on Ion Torrent company, which uses integrated circuit chips to directly convert chemical signals into digital signals. Ion Torrent's design is based on the sensing of accumulated charge to the change of the field-effect transistor (FET) threshold voltage. However, since the charges (hydrogen ions) generated during the sequencing process, the charge cannot be completely removed even when the wafer is cleaned regularly with the buffer solution. Noise increase is due to the accumulation of charge in the wafer well, the DNA sequence can only be read with a length of approximately 200 base pairs (bp). Furthermore, when the technology is downsizing the wafer process, if the wafer well has charge remaining, it will be more easily to produce noise.

Therefore, how to provide a faster and more accurate DNA sequencing chip and method, the existing technology needs to be improved.

### SUMMARY

The disclosure provides an element and biochip for nucleotide sequencing and sequencing analysis method thereof, thereby removing the charges generated during sequencing and avoiding the increase of noise caused by charge accumulation.

One aspect of the present disclosure provides a nucleotide sequencing element, comprising: a substrate; a transistor disposed on the substrate; a dielectric layer covering the transistor; a circular electrode set located on the dielectric layer and having an opening exposed the dielectric layer, and the circular electrode set and the dielectric layer forming a well, wherein the circular electrode set comprises: at least one first circular electrode; a second circular electrode located on the first circular electrode; and a third circular electrode located on the second circular electrode; and a conductor deposed in the dielectric layer, one end of the conductor connected to a source or a drain of the transistor, the other end of the conductor connected to the first circular electrode or the second circular electrode of the circular electrode set.

In some embodiment, the first circular electrode, the second circular electrode, and the third circular electrode have a circular shape, and the first, the second, and the third circular electrodes are aligned with each other.

In some embodiment, the first circular electrode, the second circular electrode, and the third circular electrode have a polygon shape, and the first, the second, and the third circular electrodes are aligned with each other.

In some embodiment, the circular electrode set further comprises: at least one first circular spacer layer located between the dielectric layer and the first circular electrode; a second circular spacer layer located between the first circular electrode and the second circular electrode; and a third circular spacer layer located between the second circular electrode and the third circular electrode.

In some embodiment, the nucleotide sequencing element further comprises a plurality of first circular electrodes and a plurality of first circular spacer layers, wherein the first circular electrodes and the first circular spacer layers are stacked on top of each other, and the first circular electrodes electrically connected to the conductor.

In some embodiment, the nucleotide sequencing element further comprises at least one protruding electrode located in the well, and the protruding electrode having a protrusion protruding from the dielectric layer and electrically connected to the conductor.

In some embodiment, the protrusion has an aspect ratio from about 0.125 to about 7.5.

In some embodiment, the protrusion protrudes from an upper surface of the dielectric layer from about 0.01 µm to about 0.5 µm.

In some embodiment, the nucleotide sequencing element further comprises two to twenty protruding electrodes.

Another aspect of the present disclosure provides a nucleotide sequencing chip, comprising: a plurality of nucleotide sequencing element as above mentioned; and a sense amplifier connected to the source or the drain of each of the transistors.

In some embodiment, the nucleotide sequencing elements comprise at least one first nucleotide sequencing element and at least one second nucleotide sequencing element, the first nucleotide sequencing element is used for sequencing unknown nucleotide, the second nucleotide sequencing element is used for sequencing known nucleotide.

Another aspect of the present disclosure provides a method for sequencing analysis, comprising: providing the nucleotide sequencing chip as above mentioned, the nucleotide sequencing chip comprising at least one first nucleotide sequencing element and at least one second nucleotide sequencing element; applying a current to the circular electrode sets of the first and the second nucleotide sequencing elements; mixing at least one first carrier and at least one unknown nucleotide sequence fragment, the first carrier comprising at least one first primer binding to the unknown nucleotide sequence fragment; placing the bound first carrier and the unknown nucleotide sequence fragment in the well of the first nucleotide sequencing element; placing a second carrier in the well of the second nucleotide sequencing element, the second carrier comprising at least one second primer; adding a solution comprising polymerase and deoxyribonucleoside triphosphate to the wells of the first and the second nucleotide sequencing elements, wherein the deoxyribonucleoside triphosphate and the unknown nucleotide sequence fragment are polymerized in the well of the first nucleotide sequencing element to produce hydrogen ions and a first signal, and the deoxyribonucleoside triphosphate and the second primer are polymerized in the well of the second nucleotide sequencing element to produce hydrogen ions and a second signal, wherein the current of the circular electrode sets converts the hydrogen ions into hydrogen molecules; and using the transistors of the first and the second nucleotide sequencing elements to read the first signal and the second signal to obtain sequencing analysis results.

In some embodiment, the solution further comprises sodium hydroxide, disodium sulfate, potassium ferricyanide or a combination thereof.

In some embodiment, each of the first carriers comprises a bead and a plurality of first primers.

In some embodiment, each of the second carriers comprises a bead and a plurality of second primers being a known nucleotide sequence.

### BRIEF DESCRIPTION OF THE DRAWINGS

The disclosure can be more fully understood by reading the following detailed description of the embodiment, with reference made to the accompanying drawings as follows:
Fig. 1 is a partial perspective view of a circular electrode set of a nucleotide sequencing element according to one embodiment of the present disclosure.
Fig. 2 is a cross-sectional view of the circular electrode set of the nucleotide sequencing element according to one embodiment of the present disclosure.
Fig. 3 is a cross-sectional view of the circular electrode set of the nucleotide sequencing element according to another embodiment of the present disclosure.
Fig. 4 depicts a partial circuit diagram of the nucleotide sequencing element according to Fig. 2 of the present disclosure.
Fig. 5 depicts a partial circuit diagram of the nucleotide sequencing chip according to one embodiment of the present disclosure.

Marks in the figures are described as follows:
100, 100" nucleotide sequencing element
100A first nucleotide sequencing element
100B second nucleotide sequencing element
100C third nucleotide sequencing element
110 substrate
120, 120A, 120B, 120C transistor
121 gate
122 source
123 drain
130 dielectric layer
131 upper surface
132 lower surface
140, 140A, 140B, 140C circular electrode set
141, 141A, 141B, 141C well
142 first circular spacer layer
143 first circular electrode
144 second circular spacer layer
145 second circular electrode
146 third circular spacer layer
147 third circular electrode
150 conductor
160 protruding electrode
210 first electric double layer capacitor
220 first circular electrode charge transfer resistance
230 second circular electrode charge transfer resistance
240 third electric double layer capacitor
250 third circular electrode charge transfer resistance
260 first solution resistance
270 second solution resistance
280 word line
300 sense amplifier
310 differential sense amplifier
320 latch type sense amplifier
400 nucleotide sequencing chip
A1 first primer
A2 first primer
B1 second primer
B2 second primer
D1 width
D2 width
E1 first signal
E2 first signal
F1 second signal
F2 second signal
H1 height
P1 unknown nucleotide sequence fragment
P2 unknown nucleotide sequence fragment
U1 adaptor
U2 adaptor
X1 first carrier
X2 first carrier
Y1 second carrier
Y2 second carrier

### DETAILED DESCRIPTION

The following disclosure provides detailed description of many different embodiments, or examples, for implementing different features of the provided subject matter. These are, of course, merely examples and are not intended to limit the disclosure but to illustrate it. In addition, various embodiments disclosed below may combine or substitute one embodiment with another, and may have additional embodiments in addition to those described below in a beneficial way without further description or explanation.

Further, spatially relative terms, such as "beneath," "over" and the like, may be used herein for ease of description to describe one element or feature's relationship to another element(s) or feature(s) as illustrated in the figures. The spatially relative terms are intended to encompass different orientations of the device in use or operation in addition to the orientation depicted in the figures. The apparatus may be otherwise oriented (rotated 90 degrees or at other orientations) and the spatially relative descriptors used herein may likewise be interpreted accordingly.

The terminology used herein is for the purpose of describing particular embodiments only and is not intended to be limiting of the disclosure. As used herein, the singular forms "a", "an" and "the" are intended to include the plural forms as well, unless the context clearly indicates otherwise. It will be further understood that the terms "comprises" and/or "comprising", or "includes" and/or "including" or "has" and/or "having" when used in this specification, specify the presence of stated features, regions, integers, steps, operations, elements, and/or components, but do not preclude the presence or addition of one or more other features, regions, integers, steps, operations, elements, components, and/or groups thereof.

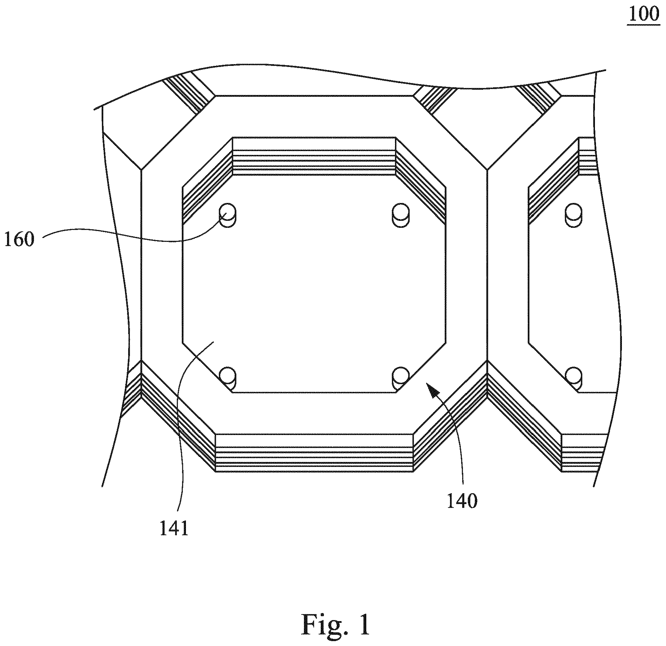

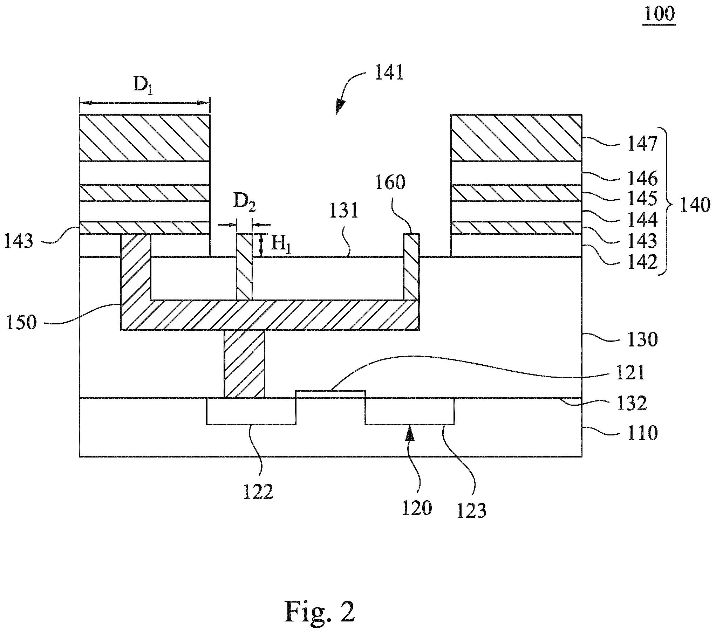

Fig. 1 is a partial perspective view of a circular electrode set of a nucleotide sequencing element according to one embodiment of the present disclosure. Fig. 2 is a cross-sectional view of the circular electrode set of the nucleotide sequencing element 100 comprising a substrate 110, a transistor 120, a dielectric layer 130, a circular electrode set 140, a conductor 150, and a protruding electrode 160. In one example, the nucleotide sequencing element 100 is used for sequencing analysis.

The substrate 110 may include, but is not limited to other semiconductor materials, such as gallium nitride (GaN), silicon carbide (SiC), silicon germanium (SiGe), germanium (Ge), or a combination thereof. The substrate 110 may contain a variety of different doping configurations depending on the design requirements in the technical field.

The transistor 120 disposed on the substrate 110, and the transistor 120 comprises a gate 121, a source 122, and a drain 123. The source 122 and the drain 123 are disposed on the substrate 110, and the gate 121 is between the source 122 and drain 123. In one example, the position of the source 122 and the drain 123 can be changed.

The dielectric layer 130 covers the transistor 120, and the dielectric layer 130 has an upper surface 131 and a lower surface 132 opposite to the upper surface 131. The material of dielectric layer 130 includes, but is not limited to, oxide, nitride, oxynitride, or a combination thereof, such as silicon oxide, silicon nitride, and silicon oxynitride. The dielectric layer 130 is made of low-k material, which makes nucleotide sequencing element 100 having good insulating property. In some embodiments, the height of the dielectric layer 130 is from about 0.02 µm to about 0.25 µm, such as about 0.10 µm, about 0.15 µm, or about 0.20 µm.

The circular electrode set 140 is disposed on an upper surface 131 of the dielectric layer 130, and has an opening to expose the dielectric layer 130. The circular electrode set 140 and the dielectric layer 130 form a well 141, and the well 141 is configured to accommodate the sample solution for test. In one example, the circular electrode set 140 has a ring shape or a polygonal shape, the ring shape such as a circle or an ellipse, and the polygonal shape such as a triangle, quadrangle, pentagon, hexagon, heptagon, octagon, enneagon, decagons, hendecagon, dodecagon, or polygons that tend to be circle, etc. In one example, the width D1 of the circular electrode set 140 is from about 0.1 µm to about 0.5 µm, such as about 0.2 µm, about 0.3 µm, about 0.4 µm. In one example, about 0.2 µm.

The circular electrode set 140 includes at least one first circular spacer layer 142, at least one first circular electrode 143, a second circular spacer layer 144, a second circular electrode 145, a third circular spacer layer 146, and a third circular electrode 147. In one example, the circular electrode set 140 from bottom to top sequentially disposed the first circular spacer layer 142, the first circular electrode 143, the second circular spacer layer 144, the second circular electrode 145, the third circular spacer layer 146, and the third circular electrode 147. The first circular electrode 143, second circular electrode 145, and third circular electrode 147 have the same shape and are aligned with each other. The first circular spacer layer 142 is disposed on the dielectric layer 130. The height of the first circular electrode 143 is less than 0.1 microns; in one example, from about 0.001 µm to about 0.1 µm, such as about 0.002 µm, about 0.005 µm, about 0.01 µm, about 0.03 µm, about 0.05 µm, about 0.07 µm, or about 0.09 µm.

In some examples, the materials of the first circular spacer layer 142, second circular spacer layer 144, and third circular spacer layer 146 include, but are not limited to oxides, nitrides, oxynitrides, or a combination thereof, such as silicon oxide, silicon nitride, and silicon oxynitride; in one example, the materials are silicon nitride. In some examples, the heights of the first circular spacer layer 142, the second circular spacer layer 144, and the third circular spacer layer 146 are respectively from about 0.02 µm to about 1 µm, such as about 0.10 µm, about 0.15 µm, about 0.20 µm, about 0.50 µm or about 0.75 µm.

In some examples, the materials of the first circular electrode 143, second circular electrode 145, and third circular electrode 147 include, but are not limited to, tantalum (Ta), tantalum nitride (TaN), copper (Cu), titanium (Ti), titanium nitride (TiN), tungsten (W), titanium (Ti), nickel (Ni), silver (Ag), aluminum (Al), copper aluminum alloy (AlCu), copper aluminum silicon alloy (AlSiCu) or a combination thereof. In one example, the materials of the first circular electrode 143, second circular electrode 145, and third circular electrode 147 are titanium nitride (TiN). In one example, the first circular electrode 143 is a working electrode (WE), the second circular electrode 145 is a reference electrode (RE), and the third circular electrode 147 is a counter electrode (CE). In another example, the first circular electrode 143 and the second circular electrode 145 are interchangeable, that is, the first circular electrode 143 can be the reference electrode, and the second circular electrode 145 can be the working electrode.

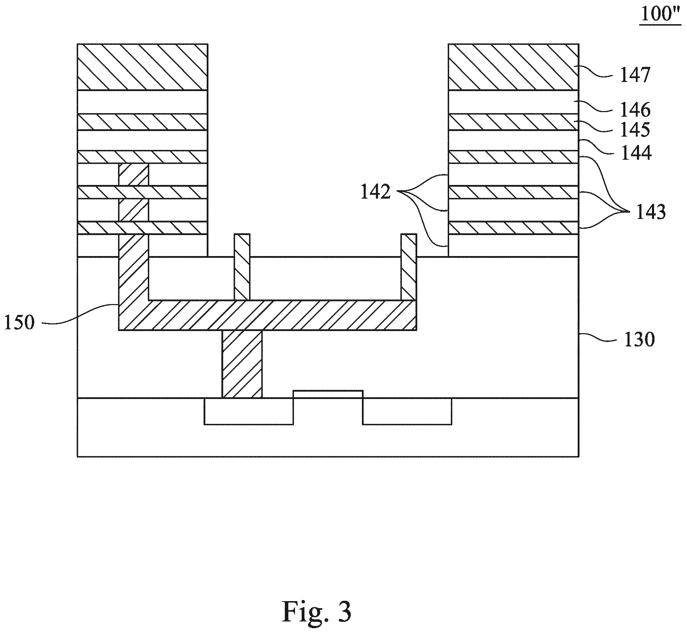

Fig. 3 is a cross-sectional view of the circular electrode set of the nucleotide sequencing element 100" according to another embodiment of the present disclosure. The number of first circular spacer layer 142 is three first circular spacer layers 142, the number of first circular electrode 143 is three first circular electrodes 143, wherein the first circular electrodes 143 and the first circular spacer layers 142 are stacked on top of each other, and the bottom first circular spacer layer 142 is disposed on the dielectric layer 130, second circular spacer layer 144 is disposed on the uppermost first circular electrode 143, wherein these first circular electrodes 143 are electrically connected to conductor 150. In one example, the first circular electrodes 143 are working electrodes.

Referring again to Fig. 2, the conductor 150 is disposed in the dielectric layer 130, one end of the conductor 150 is connected to the source 122 of the transistor 120, and the other end of the conductor 150 is connected to the first circular electrode 143 of the circular electrode set 140. Fig. 2 only shows that one end of the conductor 150 is connected to the source 122 of the transistor 120, but in other example, one end of the conductor 150 is connected to the drain 123 of the transistor 120. Fig. 2 only shows that the conductor 150 is connected to the first circular electrode 143, but in other example, the conductor 150 may be connected to the second circular electrode 145. In some examples, the material of conductor 150 includes, but is not limited to, titanium (Ti), nickel (Ni), silver (Ag), aluminum (Al), copper aluminum alloy (AlCu), copper aluminum silicon alloy (AlSiCu) or a combination thereof. In one example, the material is copper aluminum alloy.

The protruding electrode 160 is located in the well 141, and the protruding electrode 160 has a protrusion protruding from an upper surface 131 of the dielectric layer 130 to form a three-dimensional electrode. The protruding electrode 160 is equipotentially electrically connected to the conductor 150 and the first circular electrode 143, so the protruding electrode 160 and the first circular electrode 143 may have the same voltage. In one example, the first circular electrode 143 and the protruding electrode 160 are working electrodes. The protruding electrode 160 has the protrusion protruding from the dielectric layer 130. In some example, the height H1 of the protrusion is from about 0.05 µm to about 0.6 µm, such as about 0.05 µm, 0.1 µm, 0.2 µm, about 0.3 µm, or about 0.4 µm. In some example, the width D2 of the protrusion is from about 0.08 µm to about 0.4 µm, for example, about 0.08 µm, 0.1 µm, 0.2 µm, or about 0.3 µm. In another example, the protrusion has an aspect ratio from about 0.125 to about 7.5, such as about 0.2 or about 0.3. In another example, the shape of the protrusion may be a cylinder, a regular triangular column, a regular square column, a regular pentagon column, a regular hexagon column, or a regular octagon column. In some examples, the material of protruding electrode 160 includes, but is not limited to, tantalum (Ta), tantalum nitride (TaN), copper (Cu), titanium (Ti), titanium nitride (TiN), tungsten (W), titanium (Ti), nickel (Ni), silver (Ag), aluminum (Al), copper aluminum alloy (AlCu), copper aluminum silicon alloy (AlSiCu) or a combination thereof. In one example, the material of protruding electrode 160 is titanium nitride (TiN). In some examples, there are a plurality of the protruding electrode 160, such as 1, 2, 3, 4, 5, 6, 7, 8, 9, 10, 10, 11, 12, 13, 14, 20, 50, or 100 protruding electrodes 160, etc. In one example, 6 protruding electrodes 160 are arranged in a ring shape with equal distance.

Specifically, when a voltage is applied to the protruding electrode 160, the protruding electrode 160 will generate an electric field around the protrusion of the protruding electrode 160. The coverage of the electric field is not only be limited to the top surface of the protrusion, but also extends to the side wall of the protrusion, so that the electrochemical reaction is greatly increased, thereby increasing the signal strength. With the same voltage applied, the protruding electrode 160 with a three-dimensional structure provides better sensitivity than conventional planar working electrodes. It should be noted that the aspect ratio (height-to-width ratio) of the protrusion is about 0.125 to about 7.5. When the aspect ratio of the protrusion is greater than 7.5, it is easy to cause defects in the structure of the working electrode and reduce the reliability of the overall device. In one embodiment, the cylindrical protruding electrode 160 has a radius from 0.1 µm to 0.5 µm, for example, the radius is 0.15 µm, 0.2 µm, 0.25 µm, 0.3 µm, 0.35 µm, 0.4 µm, 0.45 µm, or any value between any two of these values.

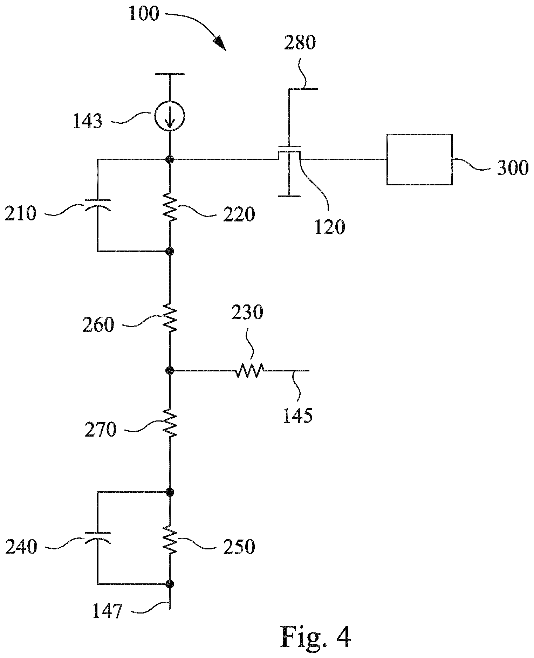

Fig. 4 depicts a partial circuit diagram corresponding to the nucleotide sequencing element 100 of Fig. 2. Please refer to Figs. 2 and 4 at the same time, the first circular electrode 143 is the working electrode, the second circular electrode 145 is the reference electrode, and the third circular electrode 147 is the counter electrode. A predetermined current is applied among the first circular electrode 143, the first circular electrode 143 and the sample solution in well 141 to form a first electric double layer capacitor 210 and a first circular electrode charge transfer resistance 220, and between the third circular electrode 147 and the sample solution in well 141 to form a third electric double layer capacitor 240 and a third circular electrode charge transfer resistance 250. The second circular electrode 145 is grounded, and a second circular electrode charge transfer resistance 230 is formed between the second circular electrode 145 and the sample solution in well 141. When current flows from the first circular electrode 143 to the third circular electrode 147 through the sample solution in well 141, a first solution resistance 260 is formed at the sample solution near the first circular electrode 143, and a second solution resistance 270 is formed at the solution near the third circular electrode 147. A word line 280 provides voltage control to enable or disable the transistor 120, which depends on requirements to control the current signal flow to the sense amplifier 300.

In some embodiments, in the nucleotide sequencing element 100 of the present disclosure, the size of the well 141 surrounded by the dielectric layer 130 and the circular electrode set 140 can be, but is not limited to any size according to the downsizing process. In one example, the size of well 141 is about 0.5 x 0.5 x 1.6 cubic micrometers (µm³) to 5.0 x 5.0 x 1.6µm³, such as 1.2 x 1.2 x 1.6 µm³, 1.5 x 1.5 x 1.6 µm³, 2.0 x 2.0 x 1.6 µm³, 3.0 x 3.0 x 1.6 µm³.

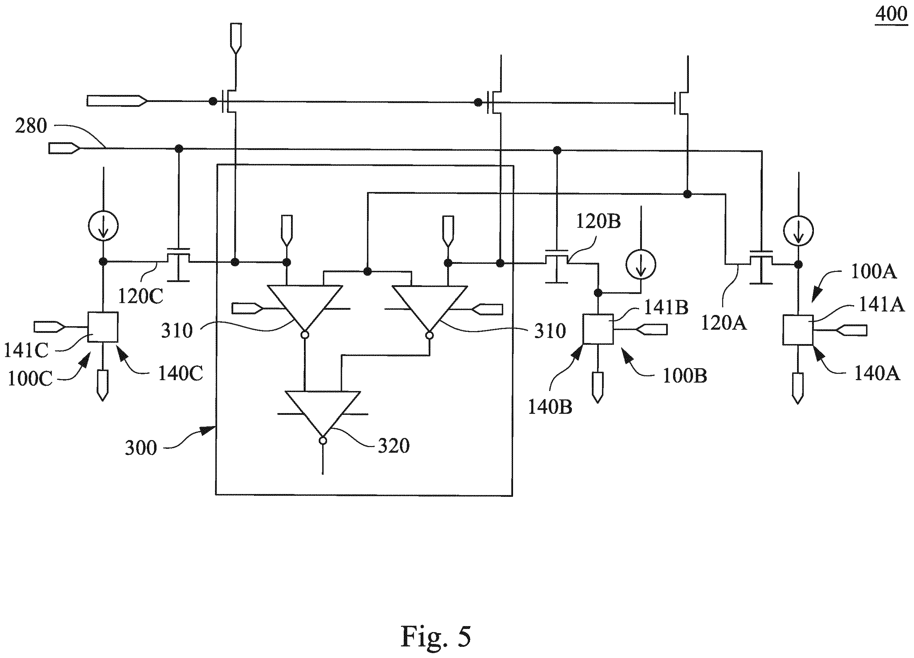

The present disclosure also provides a nucleotide sequencing chip 400. Fig. 5 depicts a partial circuit diagram of nucleotide sequencing chip 400. The nucleotide sequencing chip 400 includes a plurality of nucleotide sequencing elements and a sense amplifier 300. The sense amplifier 300 is connected to the transistors 120A, 120B, and 120C. These nucleotide sequencing elements can be divided into at least one first nucleotide sequencing element 100A, at least one second nucleotide sequencing element 100B and at least one third nucleotide sequencing element 100C. The first nucleotide sequencing element 100A is used for the sequencing of the unknown nucleotides. The second nucleotide sequencing element 100B and the third nucleotide sequencing element 100C are control groups, which provide the reference voltage value by sequencing the known nucleotide sequence. Please refer to Fig. 1 and Fig. 2. In one example, each of the first circular electrodes 143 (working electrodes) in the nucleotide sequencing elements 100 are not connected to each other, and each of the second circular electrodes 145 (reference electrodes) are connected to each other, and each of the third circular electrodes 147 (counter electrodes) are electrically connected to each other. Please refer to Fig. 5. In one example, the arrangement and addressing methods of the multiple nucleotide sequencing elements of the nucleotide sequencing chip 400 can use the related technology of the existing memory chip. Only small signals (that is, a small amount of unknown nucleotides) need to be generated during the sequential polymerization reaction, and sufficient signals can be obtained after amplification by the sense amplifier 300.

The sense amplifier 300 is a current mode, which amplifies the current signal. In one example, the nucleotide sequencing chip 400 is formed a detection matrix by a plurality of nucleotide sequencing elements 100, and the size of the detection matrix is adjusted according to the number of genes under test. In one example, the matrix size is from 0.1K x 0.1K to 100K x 100K, such as 1K x 1K, 3K x 3K, 5K x 5K, 8K x 8K, 10K x 10K, or 50K x 50K. In example, the address signal of nucleotide sequencing element 100 is controlled by 13 rows, 9 columns and 16 output devices. The row select register can read a row of nucleotide sequencing element 100 at the same time to drive the signal voltage to a column. The column select register selects one of the columns to output the signal to the sense amplifier 300, the word line 280 is given a pulse to detect the column, and an ISO signal is generated before the word line 280 is off.

The sense amplifier 300 amplifies the signals outputting from each of the nucleotide sequencing elements 100, and the voltage differences between the electrochemical reactions performed in different nucleotide sequencing elements 100 was compared. In one example, a biosensing chip has two differential sense amplifiers 310, one latch type sense amplifier 320, three p-channel metal-oxide-semiconductor equalization (PMOS equalization), and the first nucleotide sequencing element 100A, the second nucleotide sequencing element 100B and the third nucleotide sequencing element 100C.

The present disclosure also provides a method for sequencing analysis.

Although a series of operations or steps are used below to describe the method disclosed herein, an order of these operations or steps should not be construed as a limitation to the present disclosure. For example, some operations or steps may be performed in a different order and/or other steps may be performed at the same time. In addition, all shown operations, steps and/or features are not required to be executed to implement an embodiment of the present disclosure. In addition, each operation or step described herein may include a plurality of sub-steps or actions.

The present disclosure also provides a method for sequencing analysis, comprising the following steps.
(1) Providing nucleotide sequencing chip 400, comprising a plurality of first nucleotide sequencing elements 100A and a plurality of second nucleotide sequencing elements 100B.
(2) Applying a current to circular electrode sets 140A of the first nucleotide sequencing elements 100A and a current to circular electrode sets 140B of the second nucleotide sequencing elements 100B.
(3) Providing a plurality of first carriers and a plurality of unknown nucleotide sequence fragments. For example, the first carriers included a first carrier X1, a first carrier X2, etc., wherein the first carrier X1 included a magnetic bead and a plurality of first primers A1, the first carrier X2 included a magnetic bead and a plurality of first primers A2, and so forth. The unknown nucleotide sequence fragments were fragmented below 2000K bp, for example, 1900K bp, 1700K bp, 1600K bp, 1500K bp, 1300K bp, 1000K bp, 500K bp, 100K bp, 50K bp, 10K bp, 5K bp, 3K bp, 1K bp, 0.1K bp, or 0.01K bp. The unknown nucleotide sequence fragments can further be divided to, such as, unknown nucleotide sequence fragment P1, unknown nucleotide sequence fragment P2, etc. Adaptors U1 were connected to two ends of the unknown nucleotide sequence fragment P1, adaptors U2 were connected to two ends of the unknown nucleotide sequence fragment P2, and so forth.
(4) Mixing the first carriers and the unknown nucleotide sequence fragments, the first primers A1 located on the first carrier X1 specifically identified and bound to the adapters U1 at both ends of the unknown nucleotide sequence fragment P1, and the first primers A2 located on the first carrier X2 specifically identified and bound to the adapters U2 at both ends of the unknown nucleotide sequence fragment P2. In the present disclosure, "adaptor" refers to a specific nucleotide sequence with a size of about 10 bp to 100 bp, which can be complementary and bind to the first primer. In one example, after the first primers A1 were bound to the unknown nucleotide sequence fragments P1, the unknown nucleotide sequence fragments P1 were amplified by the polymerase chain reaction (PCR) to obtain a plurality of unknown nucleotide sequence fragments P1. And then, each of the unknown nucleotide sequence fragments P1 were bound to other first primers A1 on the first carrier X1, so that the first carrier X1 had a plurality of the same unknown nucleotide sequence fragments P1, and so forth.
(5) Placing the bound first carrier X1 and the unknown nucleotide sequence fragments P1 in the well 141A of the first nucleotide sequencing element 100A. The bound first carrier X2 and unknown nucleotide sequence fragment P2 were placed in the well 141A of another first nucleotide sequencing element 100A, and so on. In other words, there is only one first carrier in well 141A of the first nucleotide sequencing element 100A.
(6) Providing a plurality of second carriers, for example, the second carriers included second carrier Y1, second carrier Y2, and so forth. The second carrier Y1 included a magnetic bead and a plurality of second primer B1, each of the second primers B1 included one adenine. Second carrier Y2 included a magnetic bead and a plurality of second primers B2, the second primers B2 included three consecutive adenines. That is, the sequences of the second primers on the same second carrier are the same. In one example, the second primer was a known synthetic nucleotide sequence including, but was not limited to single adenine (A), single thymine (T), single cytosine (C), single guanine (G), single uracil (U), or multiple repeating adenines (A), multiple repeating thymines (T), multiple repeating cytosines (C), multiple repeating guanines (G), multiple repeated uracils (U), such as AAA, TTT, CCC, GGG, or UUU.
(7) Placing each second carrier in the well 141B of each second nucleotide sequencing element 100B, that is, one second carrier was placed in one well 141B of the second nucleotide sequencing element 100B.
(8) Adding a solution containing polymerase and deoxyribonucleoside triphosphate (dNTP) to the well 141A of each first nucleotide sequencing element 100A and the well 141B of each second nucleotide sequencing element 100B. Deoxyribonucleoside triphosphate and the unknown nucleotide sequence fragment in well 141A of the first nucleotide sequencing element 100A were polymerized to produce hydrogen ions and a first signal. For example, the first nucleotide sequencing element 100A loaded with first carrier X1 and the unknown nucleotide sequence fragment P1 generated a first signal E1, and the first nucleotide sequencing element 100A loaded with first carrier X2 and the unknown nucleotide sequence fragment P2 generated a first signal E2. On the other hand, deoxyribonucleoside triphosphate and the second primer in well 141B of the second nucleotide sequencing element 100B were polymerized to generate hydrogen ions and a second signal. For example, the second nucleotide sequencing element 100B loaded with second carrier Y1 generated a second signal F1, and the second nucleotide sequencing element 100B loaded with second carrier Y2 generated a second signal F2. In the above-mentioned polymerization reaction, the generated hydrogen ions were converted into hydrogen molecules by the current applied to the circular electrode sets 140A, 140B.
   In one example, the solution included pure water, sodium hydroxide, polymerase, magnesium salt, disodium sulfate and potassium ferricyanide, and the pH value was alkaline to provide substances required for the polymerization reaction. Disodium sulfate and potassium ferricyanide were the media to assist the redox in the electrochemical reaction, to enhance the amount of current change, and to make the signal easier to detect.
(9) Using the transistors 120A of the first nucleotide sequencing elements 100A and the transistors 120B of the second nucleotide sequencing elements 100B to read the first signal and the second signal, and to obtain the sequencing result.

In one example, deoxyribonucleoside triphosphate was added sequentially, for example, deoxyadenosine triphosphate (dATP), deoxythymidine triphosphate (dTTP), deoxycytidine triphosphate (dCTP), and deoxyguanosine triphosphate (dGTP) to the well 141A of the first nucleotide sequencing element 100A and the well 141B of these second nucleotide sequencing element 100B, and the wells will be washed before adding the next deoxyribonucleoside triphosphate. Specifically, when deoxyribonucleoside triphosphates were complementary to the unknown nucleotide sequence fragments or the second primers, hydrogen ions (H⁺) were released and the pH value decrease, so that the impedance value was decrease. As the impedance value decrease, the current flowing through the circular electrode sets 140A, 140B increased, and the current flowing through the transistors 120A, 120B decreased, thereby generating electrical signals. The hydrogen ions could be quickly neutralized with the electrons released from the working electrode to form hydrogen gas, so that there is no residual hydrogen ion in the wells 141A, 141B to affect the subsequent interpretation. Please refer to Fig. 2, since the third circular electrode 147 (counter electrode) is placed at the top of the circular electrode set 140 to form a barrier, the hydrogen ions generated during the polymerization cannot cross the third circular electrode 147 to the next well 141, so that the sequencing process is more accurate.

In one example, please refer to Fig. 5, the first nucleotide sequencing element 100A was washed after the polymerization, then the solution was added and mixed again to perform polymerization, and then this step was repeated until the unknown nucleotide sequence fragments were all sequenced. In another example, the first nucleotide sequencing elements 100A that had undergone the polymerization may not be washed, then the solution was added and mixed again to perform polymerization, and then this step was repeated until the unknown nucleotide sequence fragments were all sequenced.

Please refer to Fig. 5 again, the first signal of the first nucleotide sequencing element 100A is respectively amplified and compared with the second signal of the second nucleotide sequencing element 100B and the third signal of the third nucleotide sequencing element 100C by two differential sense amplifiers 310, and the two differential sense amplifiers 310 respectively generated and outputted two signals to a latch type sense amplifier 320 for comparison and analysis.

In one example, a second carrier was placed in the well 141B of the circular electrode set 140B of the second nucleotide sequencing element 100B, and the second primer of the second carrier comprised a single thymine (T). A third carrier was placed in the well 141C of the circular electrode set 140C of the third nucleotide sequencing element 100C, and the third primer of the third carrier comprised three thymines (TTT). When the dATP was added in the well 141A of the first nucleotide sequencing element 100A, the well 141B of second nucleotide sequencing element 100B and the well 141C of third nucleotide sequencing element 100C, single nucleotide polymerization reaction (A-T pairing) was performed in the well 141B of the second nucleotide sequencing element 100B to generate a second signal (for example, 0.3 Voltage (0.3V)). Three nucleotide polymerization reactions (three A-T pairs) were performed in the well 141C of the third nucleotide sequencing element 100C to generate a third electrical signal (for example, 1V). If the electrical signal generated from the first nucleotide sequencing element 100A is 0V, it means that no polymerization occurs; if the electrical signal is 0.3V, it means that a single nucleotide (A) was polymerized; if the electrical signal is 0.6V, it means that two nucleotides (AA) are polymerized; if the electrical signal is 1V, it means that three nucleotides (AAA) are polymerized. Specifically, the second signal and the third signal are reference voltage.

In one example, in addition to providing reference voltage values of different kinds of nucleotides (A, T, C, G), the nucleotide sequencing elements further provide reference voltages for the same nucleotide but different numbers of nucleotides values including, but are not limited to 1, 2, 3, 4, 5, 6, 7, 8, 9, 10, 15, or 20. At the beginning of sequencing, the relative reference voltage was obtained from artificially synthesized primers of known nucleotide sequences according to the environment and temperature of the wafer. Therefore, the reference voltage values of the same nucleotide and the same number of nucleotides in different batches will be slightly different because of the current environment, so as to more accurately determine the sequence of the nucleotide under test.

The advantages of the present disclosure are that:
The working electrode provides electrons in the electrochemical reaction, and the hydrogen ions generated in the polymerization reaction are neutralized to generate hydrogen gas, thereby effectively removing the charges generated during sequencing and avoiding the increase of noise caused by charge accumulation.

Since the electrochemical reaction is fast, the next nucleotide sequence can be performed after the hydrogen ions are immediately removed, so the overall sequencing time can be shortened.

Because the hydrogen ions in the well can be effectively removed, DNA fragments up to 2000K bp can be read in the same well.

Metal oxide semiconductor technology is used to reduce the cost of sequencing equipment, and large-scale production and downsizing process are performed to obtain higher density and larger array size.

When the process is downsized, the volume of the well on the nucleotide sequencing element is smaller. Once the polymerization reaction and the electrochemical reaction occur, the pH value will change dramatically to shorten the detection time, reduce the number of unknown nucleotide sequence fragments, reduce reagent consumption, and thus reduce costs.

Since the reference voltage value of each batch will be slightly different because of the current environment, the relative reference voltage can be obtained by artificially synthesizing known nucleotide sequence primers, so that the sequence of unknown nucleotide sequence fragment can be more accurately determined.

While the disclosure has been described by way of example(s) and in terms of the preferred embodiment(s), it is to be understood that the disclosure is not limited thereto. On the contrary, it is intended to cover various modifications and similar arrangements and procedures, and the scope of the appended claims therefore should be accorded the broadest interpretation so as to encompass all such modifications and similar arrangements and procedures.

## Claims

1. A nucleotide sequencing element, comprising:
a substrate;
a transistor disposed on the substrate;
a dielectric layer covering the transistor;
a circular electrode set located on the dielectric layer and having an opening exposed the dielectric layer, and the circular electrode set and the dielectric layer forming a well, wherein the circular electrode set comprises:
at least one first circular electrode;
a second circular electrode located on the first circular electrode; and
a third circular electrode located on the second circular electrode; and
a conductor deposed in the dielectric layer, one end of the conductor connected to a source or a drain of the transistor, the other end of the conductor connected to the first circular electrode or the second circular electrode of the circular electrode set.

2. The nucleotide sequencing element of claim 1, wherein the first circular electrode, the second circular electrode, and the third circular electrode have a circular shape, and the first, the second, and the third circular electrodes are aligned with each other.

3. The nucleotide sequencing element of claim 1, wherein the first circular electrode, the second circular electrode, and the third circular electrode have a polygon shape, and the first, the second, and the third circular electrodes are aligned with each other.

4. The nucleotide sequencing element of claim 1, wherein the circular electrode set further comprises:
at least one first circular spacer layer located between the dielectric layer and the first circular electrode;
a second circular spacer layer located between the first circular electrode and the second circular electrode; and
a third circular spacer layer located between the second circular electrode and the third circular electrode.

5. The nucleotide sequencing element of claim 4, further comprising a plurality of first circular electrodes and a plurality of first circular spacer layers,
wherein the first circular electrodes and the first circular spacer layers are stacked on top of each other, and the first circular electrodes electrically connected to the conductor.

6. The nucleotide sequencing element of claim 1, further comprising:
at least one protruding electrode located in the well, and the protruding electrode having a protrusion protruding from the dielectric layer and electrically connected to the conductor.

7. The nucleotide sequencing element of claim 6, wherein the protrusion has an aspect ratio from about 0.125 to about 7.5.

8. The nucleotide sequencing element of claim 6, wherein the protrusion protrudes from an upper surface of the dielectric layer from about 0.01 µm to about 0.5 µm.

9. The nucleotide sequencing element of claim 6, further comprising two to twenty protruding electrodes.

10. A nucleotide sequencing chip, comprising:
a plurality of nucleotide sequencing element as claimed in claim 1; and
a sense amplifier connected to the source or the drain of each of the transistors.

11. The nucleotide sequencing chip of claim 10, wherein the nucleotide sequencing elements comprise at least one first nucleotide sequencing element and at least one second nucleotide sequencing element, the first nucleotide sequencing element is used for sequencing unknown nucleotide, the second nucleotide sequencing element is used for sequencing known nucleotide.

12. A method for sequencing analysis, comprising:
providing the nucleotide sequencing chip as claimed in claim 10, the nucleotide sequencing chip comprising at least one first nucleotide sequencing element and at least one second nucleotide sequencing element;
applying a current to the circular electrode sets of the first and the second nucleotide sequencing elements;
mixing at least one first carrier and at least one unknown nucleotide sequence fragment, the first carrier comprising at least one first primer binding to the unknown nucleotide sequence fragment;
placing the bound first carrier and the unknown nucleotide sequence fragment in the well of the first nucleotide sequencing element;
placing a second carrier in the well of the second nucleotide sequencing element, the second carrier comprising at least one second primer;
adding a solution comprising polymerase and deoxyribonucleoside triphosphate to the wells of the first and the second nucleotide sequencing elements, wherein the deoxyribonucleoside triphosphate and the unknown nucleotide sequence fragment are polymerized in the well of the first nucleotide sequencing element to produce hydrogen ions and a first signal, and the deoxyribonucleoside triphosphate and the second primer are polymerized in the well of the second nucleotide sequencing element to produce hydrogen ions and a second signal, wherein the current of the circular electrode sets converts the hydrogen ions into hydrogen molecules; and
using the transistors of the first and the second nucleotide sequencing elements to read the first signal and the second signal to obtain sequencing analysis results.

13. The method of claim 12, wherein the solution further comprises sodium hydroxide, disodium sulfate, potassium ferricyanide or a combination thereof.

14. The method of claim 12, wherein each of the first carriers comprises a bead and a plurality of first primers.

15. The method of claim 12, wherein each of the second carriers comprises a bead and a plurality of second primers being a known nucleotide sequence.
